# EUROPEAN PATENT APPLICATION

(11) **EP 1 281 769 A1**
(43) Date of publication of application: **05.02.2003**
(21) Application number: 01402085.3
(22) Date of filing: 01.08.2001
(51) Int. Cl.: C12Q 1/42, C12Q 1/18, C12Q 1/48, C12Q 1/527

(54) **Screening-method for inhibitors of folate-synthesis-pathway enzymes**

(71) Applicant: Aventis Pharma S.A., 92165 Antony Cédex (FR)
(72) Inventor: Stachyra, Thérèse, 94350 Villiers sur Marne (FR); Biton, Jacques, 60610 Lacroix Saint Ouen (FR)
(74) Representative: Pochart, François

(57) **Abstract**

The invention is directed to a method for determining the inhibition activity of a compond to be tested on at least the DHPS activity of the folate synthesis pathway, which is coupled with the detection of the release of formed inorganic phosphate. An enzymatic assay, implementing this method of determination on an enzymatic composition, is also within the scope of the invention.

This assay is suitable for high throughput screening of compounds affecting folate pathway.

## Description

### FIELD OF THE INVENTION

The invention is directed to a method for determining the inhibition activity of a compound to be tested on at least the DHPS activity of the folate synthesis pathway, which is coupled with the detection of the release of formed phosphate.

### BACKGROUND OF THE INVENTION

Reduced folate cofactors are essential for the synthesis of purines, thymidilate, glycine methionine, panthotenic acid and N-formyl methionyl-tRNA. Folates are vitamins for humans while most microbial cells must synthesize folates *de novo* since they lack the carrier mediated active transport system of mammalian cells that allows the use of preformed dietary folates.

In *P.carinii* (Volpe et al 1995), *S.cerevisiae* (Sen-Gupta et al., 1997), and *C.albicans,* one gene encodes for three of the enzymes of the folate de novo pathway. The *fol 1* gene from *C.albicans* codes for a putative protein of 88.6kDa (788 amino acids), see PCT Application WO00/15838 (CaNL 256), see SEQ ID 1.

The Fol 1 protein sequences show significant homology with proteins to be involved in the biosynthesis of dihydropteroate (Lopez et al., 1993, Slock et al., 1990) in both eukaryotic and prokaryotic microorganisms.

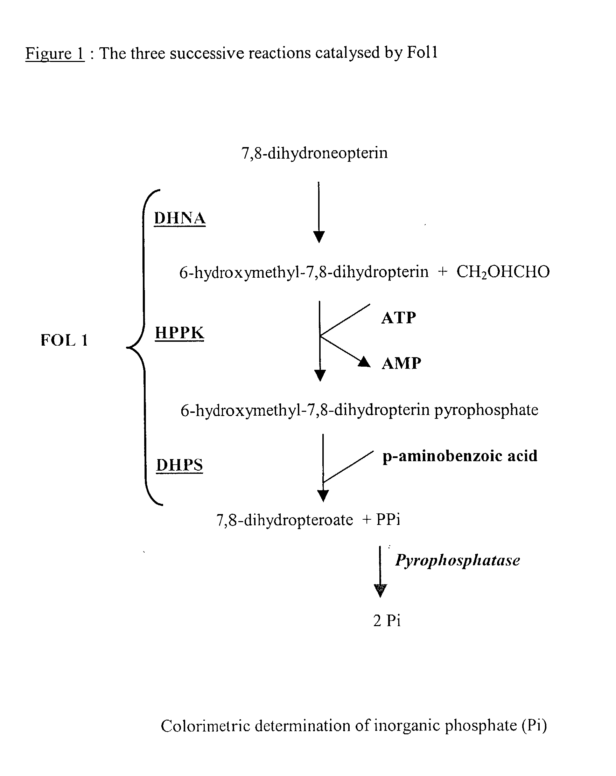

The *fol 1* gene from *P.carinii*, and *S. cerevisiae* and *C.albicans* encodes a multifunctional enzyme that catalyses the last three consecutive steps of the 7,8-dihydropteroate biosynthesis pathway (see Figure 1). The first activity is the 7,8-dihydroneopterin aldolase or DHNA which catalyses the conversion of 7,8-dihydroneopterin to 6-hydroxymethyl-7,8-dihydropterin. The second activity is the 7,8-dihydro-6-hydroxymethylpterin-pyrophosphokinase or HPPK; it converts the product of DHNA reaction to 6-hydroxymethyl-7,8-dihydropterin pyrophosphate. The third activity is the dihydropteroate synthase or DHPS, it catalyses the condensation of *para*-aminobenzoic acid (pABA) with the 6-hydroxymethyl-7,8-dihydropterin pyrophosphate to form 7,8-dihydropteroate.

With no mammalian counterparts, these enzymes are attractive chemotherapeutics targets, since it is possible to target highly selective drugs against them for treating microbial diseases. A number of compounds have been developed as antimicrobial agents which target two of the seven enzymes in the folate pathway. Trimethoprim inhibits dihydrofolate reductase, thereby preventing the formation of tetrahydrofolate (Huovinen et al., 1995). The sulphonamides exert their antimicrobial effect on a different biosynthetic enzyme, dihydropteroate synthase (Slock et al., 1990).

Amino acid sequence comparisons and functional studies reveal marked diversity in DHNA, HPPK and DHPS structure and organization in the different species (Slock et al., 1990 ; Lopez et al., 1993). Depending of the source, these three activities are expressed as a mono-, bi- or trifunctional enzyme on the same polypeptide. Taking into account the difficulty of producing multifunctional enzymes, in the case of multifunctional enzymes, the approaches in the literature were to isolate and express the monofunctional domain sequences with an aim to study the enzymatic system (Volpe et al., 1995).

Previous studies with monofunctional protein require the coupling of DHNA activity with extrinsic purified HPPK and DHPS activities, or the coupling of HPPK with extrinsic purified DHPS (Lopez et al., 1993). Moreover, it also involves synthesis of the HPPK or the DHPS substrates.

Assays described in the literature (Lopez et al., 1993, Volpe et al., 1995) are not amenable to high throughput screening (H.T.S). They generally used radiolabelled substrate: para-amino benzoic acid ([³H]pABA or [¹⁴C]pABA), that involves a separation step of the substrate from the products before counting the radioactivity. Formed radioactive dihydropteroate is separated from substrates by paper chromatography and measured in a scintillation counter (Volpe et al., 1995). The radioactive dihydropteroate can be separated from unreacted [³H] pABA by the ether extraction method before measuring the radioactivity in the scintillation counter. These steps, solvent extraction or paper chromatography, are not adaptable to H.T.S.

### SUMMARY OF THE INVENTION

The invention is directed to a method for determining the inhibition activity of a compound to be tested on one of the enzymes of the folate synthesis pathway, comprising the steps of incubation of said compound with an enzymatic composition having at least the DHPS activity, and of detection of the release of phosphate.

In a preferred embodiment, the DHPS activity is coupled with pyrophosphatase.

According to one embodiment of the invention, the enzymatic composition has a DHPS activity, or DHPS and HPPK activities, or DHPS and HPPK and DHNA activities.

This method of determination involves also the use of an (the) enzyme(s) which is (are) encoded by SEQ ID N°1 or a homolog thereof and functional fragments thereof as well as by the corresponding encoded protein of SEQ ID N°1 or a functional polypeptidic fragment thereof.

According to another embodiment, the method of determination is performed on an enzymatic composition which is derived from fungi, bacteria or protozoa.

The method of determination comprises an incubation step of the compound to be tested with 7,8-dihydroneopterin, ATP, pABA, Fol1, pyrophosphatase and a detection step of the release of phosphate. This can be applied by the measurement of DHPS activity but also of the DHNA and/or HPPK activities.

This method of determination allows the *in vitro* synthesis of the HPPK substrate, DHPS substrate and product.

The preferred embodiment in the present invention is the detection of phosphate by colorimetry method. However, detection of phosphate can also be performed by fluorometry or by radioactive means.

The invention is also directed to a method of screening of compounds with potential inhibitory effect on enzymatic composition by implementing the method of determination presented above.

Finally, the invention concerns an enzymatic assay for *in vitro* testing compounds for their inhibitory effect on an enzymatic composition as disclosed earlier, comprising the substrate 7,8-dihydroneopterin, and the Foll enzyme, and the enzyme pyrophosphatase, and pABA and ATP for simultaneous or sequential use.

### BRIEF DESCRIPTION OF THE FIGURES

Fig.1 Scheme of the three reactions catalysed by Fol1.
Fig.2 Kinetics of DNHA+HPPK+DHPS, HPPK+DHPS and DHPS activities. The absorbance is measured at 850nm; time scale is in minutes.
Fig.3 In assay 1, kinetics of DNHA+HPPK+DHPS activities are measured at 850nm in samples. After one hour incubation period, the substrate 7,8-dihydroneopterin is added to the samples with or without inhibitor.
Fig.4 In assay 2, kinetics of HPPK+DHPS activities are measured at 850nm in samples. After one hour incubation period, the substrate ATP is added to the samples with or without inhibitor.
Fig.5 In assay 3, kinetics of DHPS activity are measured at 850nm in samples. After one hour incubation period, the substrate pABA is added to the samples with or without inhibitor.

### DETAILED DESCRIPTION OF THE INVENTION

The *fol1* gene from *C.albicans* coding for DHNA, HPPK and DHPS, has been cloned and expressed in *E.coli.* The recombinant protein has been purified, and the enzymatic activity of this protein has been studied.

One object of the invention is to provide a simple and rapid method to screen a large number of compounds for their capability to inhibit specifically the Fol1 enzyme by the mean of inhibition of at least one of its 3 activities (DHNA, HPPK, and DHPS).

The invention is directed to a method and an enzymatic assay which involve a multifunctional protein Fol 1, which catalyses the sequential reactions by DHNA, HPPK, and DHPS. These robotics-based automated assay for measuring DHNA, HPPK, and DHPS coupled activities by inorganic phosphate detection are then adaptable to high throughput screen (H.T.S.) that can be used to discover new antibacterial, antifungal or antiprotozoa drugs.

These method and assay have been proved versatile as they allows to target either the 3 activities DHNA, HPPK and DHPS together (trifunctional protein) or only one of these activities (monofunctional proteins). Consequently, the screening assay can be used to target antibacterial, antimicrobial or antiprotozoa enzymes.

The trifunctionality of Fol1 has been confirmed also by the HPLC and HPLC/MS methods.

Thus, this method for screening inhibitory compounds can be applied on enzymatic reactions which derive from the following organisms: bacteria, fungi or protozoa.

One preferred embodiment is directed to an enzymatic assay which is performed on recombinant Foll purified protein from *C. albicans.*

The measurement of DHNA, HPPK and DHPS activities is coupled with pyrophosphatase activity leading to the detection of inorganic phosphate. Colorimetric, fluorimetric or radioactive methods of inorganic phosphate detection can be used to detect inorganic phosphate. For example, it is possible to detect spectrophotometrically inorganic phosphate by the reaction catalyzed by uridine diphosphoglucose pyrophosporylase and coupling to phosphoglucose mutase and glucose R-phosphate dehydrogenase (Johnson, et al., 1968). According to one embodiment, a similar enzymatic reaction with uridine 5'-diphospho[¹⁴C]glucose can be used such as disclosed in Cheung.

According to another embodiment, the fluorometric method carries out the determination of inorganic phosphate by coupling the UDPG pyrophophorylase reaction with three other enzymes in a system of phosphorylation and double reduction to form NADPH as shown in Caines.

The preferred method for this screening assay is based on a colorimetric phosphate detection avoiding the use of radiolabelled substrates of DHNA, HPPK or DHPS. In order to accomplish this, the DHPS reaction is coupled with a pyrophosphatase to perform a detection.

As the use of a radioelement is optional, the Fol1 assay is adaptable to high throughput screening (H.T.S).

A spectrophotometric assay is used to measure the release of phosphate catalyzed by the pyrophosphatase which is added to the reaction mixture. The phosphate is detected by a colorimetric method based on formation of a phosphomolybdate complex (Upson et al, 1996 ; Baykov, 1989). This spectrophotometric assay is capable of automation.

The colorimetric reaction is monitored by measuring the increase in absorbance at 850 nm. The generation of two molecules of phosphate from each molecule of pyrophosphate increases the sensitivity of the assay, which has a linear range at least from 2 to 20 nmol of pyrophosphate in a reaction volume of 200 µl.

The assay of the present application is suitable for H.T.S. by following the three reactions catalysed by Fol1 as presented hereafter. The reaction medium contains the substrates : 7,8-dihydroneopterin, ATP and pABA. In the absence of inhibitor, the 3 enzymatic activities of Fol1 have different kinetics. The inventors have shown that at 37°C, the HPPK reaction is 2 times faster than the DHNA reaction and that the DHPS reaction is about 5 times faster than the HPPK reaction and 10 times faster than the DHNA reaction. Thus, in the absence of inhibitor, the 3 enzymatic activities of Fol1 have different kinetics.

This invention further provides method and reagents for *in vitro* synthesis of the HPPK substrate, the DHPS substrate and the DHPS product. As substrates of these enzymatic activities, only the 7,8-dihydroneopterin (DHNA substrate) is commercially available, the other substrates are unstable and difficult to prepare. This method thus avoids synthesizing chemically the 6-hydroxymethyl-7,8-dihydropterin (HPPK substrate) and the 6-hydroxymethyl-7,8-dihydropterin pyrophosphate (DHPS substrate).

The enzymatic compositions for the determination of enzymatic activities according to the present invention can be obtained from crude extracts or by genetic recombination. The preferred method of preparation is via genetic recombination. These techniques are well known for the skilled man.

Compounds are tested for a potential inhibitory effect by performing the assay in three reaction conditions described hereafter. It is still within the scope of the invention to perform only 1 or 2 out of these 3 reactions, in particular when the enzymatic enzyme(s) affected by the inhibitor is determined.

The global inhibition assay compares activities of the three enzymes in three different reaction conditions. This global inhibition assay is performed in 3 different steps, referred below as assay 1, assay 2 and assay 3. Following these steps, if an inhibition is found with reactions conditions 1 but not with reactions conditions 2 and 3, the target of the inhibitor is the first enzymatic activity (i.e. DHNA). If an inhibition is found with reaction conditions 2, but not 1 and 3, the inhibitor's target is the second enzymatic activity (i.e. HPPK). If only the third assay shows inhibition with a potential inhibitor, the target is then the third activity (DHPS).

### EXAMPLES

### Assay 1: Monitoring of the combined activities DHNA and HPPK and DHPS

The global reaction is followed monitoring the release of inorganic phosphate. The whole reaction is dependent on DHNA reaction which is the limiting reaction at 37°C.

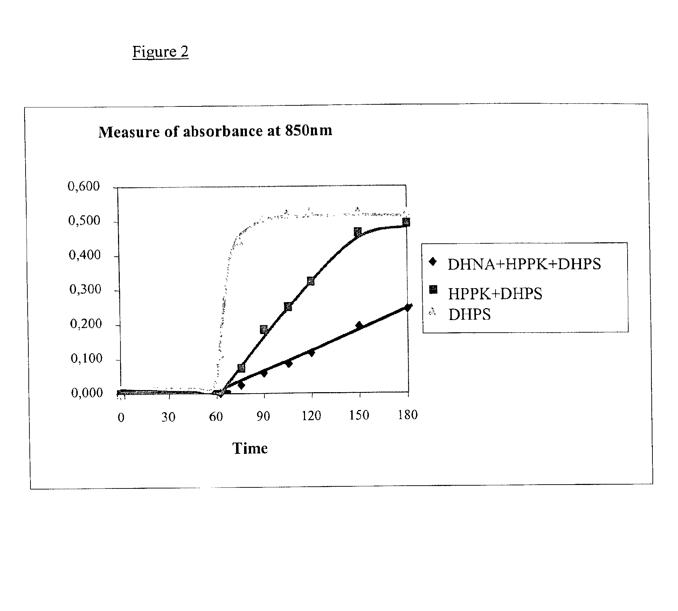

The kinetics of the Fol1 activities are given in Figure 2.

During this assay (or screening 1), the aim is to specifically find DHNA inhibitors (the slowest reaction). Total reaction medium and the putative inhibitor are used and the reaction is maintained at 37°C for 120 minutes.

According to this assay, the 3 Foll activities (i.e. DHNA, HPPK and DHPS), are measured, by adding all substrates of enzymatic reactions (7,8- dihydroneopterin, ATP and pABA) to the reaction medium containing Fol1, pyrophosphatase and the inhibitor.

The final concentrations in the reaction medium are indicated in the following chart.

| | Final concentration in the assay |
|---|---|
| Tris/HCl buffer 44 mM pH 8,2 | 40 mM |
| 2-mercaptoethanol 1 M | 10 mM |
| MgCl₂ 0,5 M | 25 mM |
| Pyrophosphatase 25 U/ml in casein 1mg/ml | 2.5 U/ml |
| ATP 10 mM | 100 µM |
| p-aminobenzoic acid 2 mM | 75 µM |
| Fol 1; 50 µg/ml in casein 1mg/ml | 5 µg/ml |
| DMSO 50% (+/- Inhibitor) | 5.00% |
| 7,8-dihydro-D-neopterin 0.5 mM | 50 µM |
| NB: The final concentration in casein is 0.2 mg/ml The reaction medium is: V_{f}=80 µl | |

### Preparation of enzymes solution stocks : Fol1 solution 10X :

An aliquot of Foll at 9.6 mg/ml is diluted first at 200 µg/ml, and once more at 50 µg/ml, in Tris buffer containing casein 1 mg/ml.

The solutions must be prepared every day and stored in ice solution.

Solution 10X of inorganic pyrophosphatase :

From a commercial flask of inorganic pyrophosphatase at 1000 U, a solution at 1000 U/ml is prepared in deionisated water (millQ). Aliquots containing 20 µl at 1000 U/ml are stored at -80°C.

One aliquot is diluted at 25 U/ml in Tris buffer containing casein 1 mg/ml. The aliquot must not be conserved more than 10 h (one day).

The reaction medium is V_{f}=80 µl.

*Pool 1 contains* : Tris buffer; 2-mercaptoethanol, MgCl₂, ATP, p-aminobenzoic acid and the Fol 1 and pyrophosphatase enzymes prepared as described previously.

The reaction medium in the 96 well Microplate contains 64 µl pool 1, then is incubated for 60 min at 37°C with shaking. This step is just to keep Fol1 in the same conditions as assay 2 and assay 3. Then, 8 µl inhibitor in DMSO 50% and 8 µl 7,8-dihydro-D-neopterin 0.5 mM are added to the reaction medium. The incubation of the reaction medium is carried out at 37°C for 1 hour with shaking.

### Stop reaction

The Foll and pyrophosphatase reactions are stopped by acidification, when the reagent 1 used for detection of inorganic phosphate is added.

### Blank (or negative control):

The reaction medium does not contain 7,8-dihydro-D-neopterin. It corresponds to the 0% activity control.

### Positive control :

The reaction medium does not contain inhibitor. It corresponds to 100% of activity.

### "False positives" control :

This control is performed to monitor the inhibition of the inorganic pyrophosphatase or the colorimetric complex.

In this control, the 50 µM of 7,8-dihydroneopterin is replaced by 50 µM of inorganic pyrophosphate in the reaction medium containing the putative inhibitor.

### • Detection of inorganic phosphate (molybdate-Arsenite method) : V_{f}=220 µl

The reagents for detection of inorganic phosphate (molybdate-Arsenite method) are the following:
Reagent 1 is composed of :
   - 6 volumes ammonium heptamolybdate 0.5% in H₂SO₄ 0.5M
   - 1 volume ascorbic acid 10%
   - 2 volumes SDS 10%
Reagent 2 is composed of :
   - sodium citrate 2%
   - sodium meta-arsenite 2%
   - acetic acid 2%

Each well contains 80 µl reaction medium. A volume of 80 µl reagent 1 is added and the mixture is incubated at 37°C for 5 min without shaking. Then, 60 µl reagent 2 is added to the mixture and the incubation is resumed at 37°C for 10 min without shaking. The absorbance is read at 850 nm using a microplate detector (Molecular Device SpectraMax Plus).

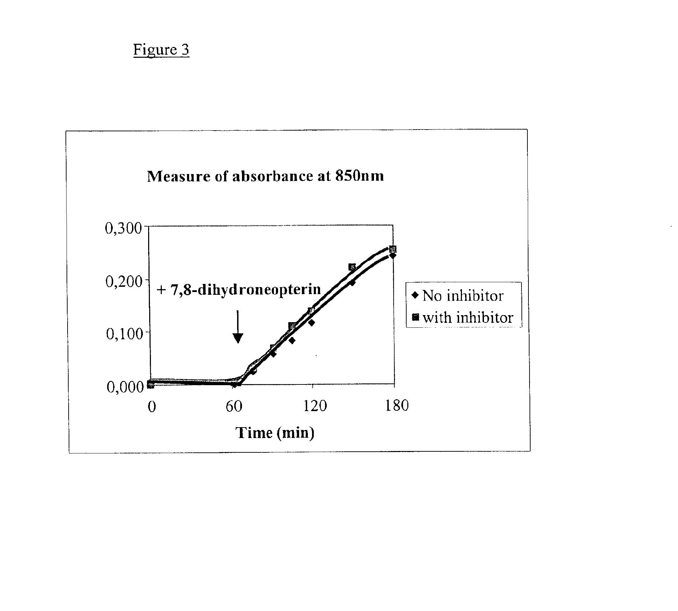

In view of the results shown in Figure 3, there is no differences in terms of activities with or without the inhibitor, therefore no inhibition is detected on combined DHNA and HPPK and DHPS activities of Fol1.

### Assay 2: Monitoring of the combined activities HPPK and DHPS

Actually, the second assay measures the inhibition of activity of HPPK since this enzyme is the limiting reaction in terms of kinetics between HPPK and DHPS. In such conditions, (same substrates and enzymes as conditions used in assay 1, ATP omitted) the product of the first enzymatic reaction (6-hydroxymethyl-7,8-dihydropterin) is accumulated in the reaction medium for a defined period of time. The final concentrations in the reaction medium are identical to those used in assay 1.

The reaction medium is V_{f}=80 µl.

*Pool 2 contains :* Tris buffer; 2-mercaptoethanol, MgCl₂, 7,8-dihydro-D-neopterin, p-aminobenzoic acid and the 2 enzymes Fol 1, Pyrophosphatase prepared as described previously.

In the 96 well Microplate is delivered 64 µl pool 2. The medium is incubated for 60 min at 37°C with shaking. During this step, the 7,8-dihydro-D-neopterin is totally converted in 6-hydroxymethyl-7,8-dihydropterin. Then, 8 µl inhibitor in DMSO 50% is added to the reaction followed by 8 µl ATP 1 mM. The reaction is carried out at 37°C for 1h with shaking.

The release of inorganic phosphate is monitored and the controls are performed as in assay 1.

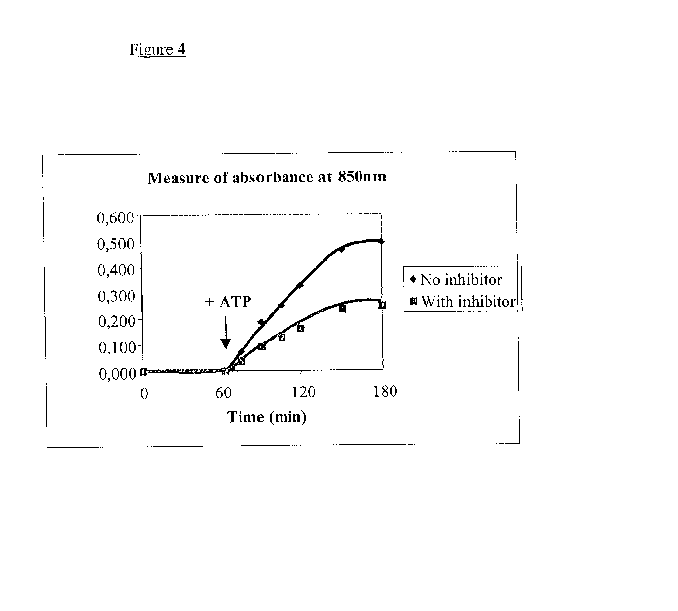

Results of the kinetics of combined HPPK and DHPS activities are shown in Figure 4.

It appears clearly from these data that 50% of inhibition is detected on combined HPPK and DHPS activities of Fol1.

### Assay 3: Monitoring DHPS activity

This assay measures the inhibition of activity of DHPS, using the same substrates and enzymes as in assay 1 without pABA.

The reaction medium is V_{f}=80 µl.

*Pool 3 contains :* Tris buffer; 2-mercaptoethanol, MgCl₂, 7,8-dihydro-D-neopterin, ATP, and the 2 enzymes Fol 1, Pyrophosphatase prepared as described previously.

In the 96 well Microplate is deliver 64 µl pool 3 and the incubation is carried out at 37°C for 60 min with shaking. During this step, 7,8-dihydroneopterin is totally converted in 6-hydroxymethyl-7,8-dihydropterin pyrophosphate. A volume of 8 µl inhibitor in DMSO 50% is added to the reaction mixture, followed by 8 µl p-aminobenzoic acid 0.75 mM. The reaction is incubated at 37°C for 1h with shaking.

The detection of the inorganic phosphate release and the controls are performed such as in assay 1.

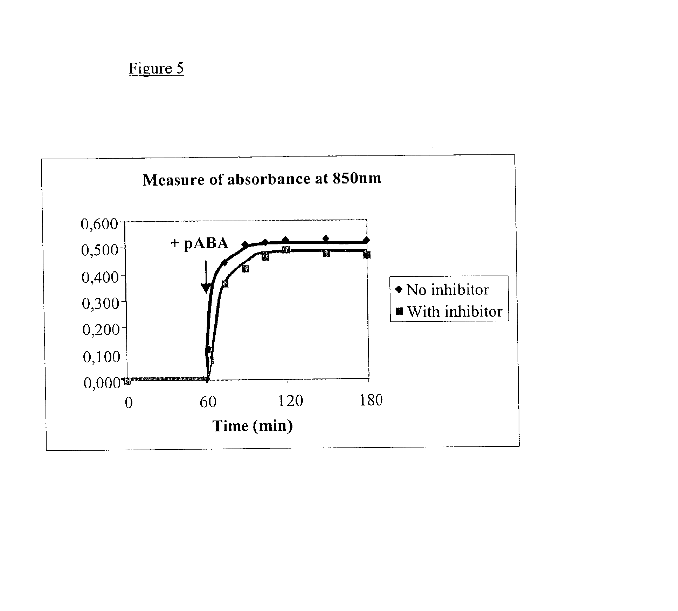

The results of kinetics of DHPS activity are presented in Figure 5.

Data show that no inhibition is detected on the DHPS activity, so the inhibitor inhibits only the HPPK activity. It has been noticed that the HPPK inhibition cannot be detected in assay 1 because in this reaction condition, the DHNA reaction is the limiting step.

### REFERENCES

**Baykov A.** Inorganic Pyrophosphate. Methods of enzymatic Analysis. Third edition, vol. VII : 558-566 **(1989).**

**Caines P.S.M., Thibert R.J., and Draisey T.F.** An improved fluorometric coupled enzymatic method for the determination of pyrophosphate in plasma and platelets: Microchemical Journal, 29, 168-181 **(1984)**

**Cheung C.P., and Suhadolnik R.J.** Analysis of inorganic pyrophosphate at the picomole level: Analytical biochemistry, 83, 61-63, **(1977).**

**Johnson J.C., Shanoff M, Bass S.T., Boezi J.A. and Hansen P.G.** An Enzymatic Method for Determination of Inorganic Pyrophosphate and Its Use as an Assay for RNA Polymerase: Anal. Biochem. 26, 137-145 **(1968)**.

**Huovinen P, Sundstrom L, Swedberg G, Skold O.** Trimethoprim and sulfonamide resistance. Antimicrob Agents Chemother. Feb;39(2):279-89 **(1995).**

**Lopez, P.; Lacks, S.A.:** A bifunctional protein in the folate biosynthetic pathway of Streptococcus pneumoniae with dihydroneopterin aldolase and hydroxymethyldihydropterin pyrophosphokinase activities: J. Bacteriol., 175; 2214-2220 **(1993)**

**Sen-Gupta M, Guldener U, Beinhauer J, Fiedler T, Hegemann JH**. Sequence analysis of the 33 kb long region between ORC5 and SUIT from the left arm of chromosome XIV from Saccharomyces cerevisiae. Yeast. Jul; 13(9) :849-60 **(1997).**

**Slock J, Stahly DP, Han CY, Six EW, Crawford IP** An apparent Bacillus subtilis folic acid biosynthetic operon containing pab, an amphibolic trpG gene, a third gene required for synthesis of para-aminobenzoic acid, and the dihydropteroate synthase gene. *J Bacterial;* 172, 12, 7211-26 **(1990).**

**Upson, R.H., Haugland, R.P., Malekzadeh, M.N.:** A spectrophotometric method to measure enzymatic activity in reaction that generate inorganic pyrophosphate. Analytical Biochemistry, 243, 1, 41-5 **(1996).**

**Volpe, F.; Ballantine, S.P.; Delves, C.J.:** Two domains with amino-acid sequence similarity are required for dihydroneopterin aldolase function in the multifunctional folic acid synthesis Fas protein of Pneumocystis carnii: Gene, 160; 41-46 **(1995)**

## Claims

1. A method for determining the inhibition activity of a compound to be tested on one of the enzymes of the folate synthesis pathway, comprising the steps of :
(i) incubating said compound with an enzymatic composition having at least the DHPS activity,
(ii)detecting the release of phosphate.

2. The method of claim 1, in which the enzyme is coupled with pyrophosphatase, step (ii) being carried out on the inorganic phosphate.

3. The method of claim 1 or 2, in which the enzymatic composition has a DHPS activity, or DHPS and HPPK activities, or DHPS and HPPK and DHNA activities.

4. The method of any of claims 1 to 3, in which the enzymatic composition is Fol1.

5. Method of determination according to any of claims 1 to 4 in which the said enzyme(s) is (are) encoded by SEQ ID N°1 or a homolog thereof and functional fragments thereof as well as by the corresponding encoded protein of SEQ ID N°1 or a functional polypeptidic fragment thereof.

6. Method of determination according to any of claims 1 to 5, in which the enzymatic composition is derived from fungi, bacteria or protozoa.

7. Method of determination according to any of claims 1 to 6, which comprises incubating the compound to be tested with 7,8-dihydroneopterin, ATP, pABA, Fol1, pyrophosphatase and detecting the release of inorganic phosphate.

8. Method of determination according to any of claims 1 to 7, in which the DHNA activity is measured, comprising the incubation of the compound to be tested with 7,8-dihydroneopterin, ATP, pABA, Fol1, pyrophosphatase and the detection of the release of inorganic phosphate.

9. Method of determination according to any of claims 1 to 8 in which the HPPK activity is measured in one single assay by :
(i) adding 7,8-dihydroneopterin, pABA but with omission of ATP to the reaction medium containing Fol1, pyrophosphate;
(ii) adding the said compound to be tested and ATP when the product of the DHNA reaction, which is 6-hydroxymethyl-7,8-dihydropterin, is accumulated after a defined period of time ;
(iii)monitoring the release of inorganic phosphate.

10. Method of determination according to any of claims 1 to 8 in which the DHPS activity is measured in one single assay by :
(i) adding 7,8-dihydroneopterin, ATP but with omission of pABA to the reaction medium containing Fol1, pyrophosphate ;
(ii) adding the said compound to be tested and pABA when the substrate of DHPS reaction, which is 6-hydroxymethyl-7,8-dihydropterin pyrophosphate, is accumulated after a defined period of time ;
(iii)monitoring the release of inorganic phosphate.

11. Method of determination according to any of claims 1 to 10, in which the DHPS substrate is synthesized *in vitro.*

12. Method of determination according to any of claims 1 to 11, in which the said detection of phosphate is performed by colorimetry.

13. Method of determination according to any of claims 1 to 11, in which the said detection of phosphate is performed by fluorometry.

14. Method of determination according to any of claim 1 to 11, in which the said detection of phosphate is performed by radioactive means.

15. Method of screening of compounds with potential inhibitory effect on enzymatic composition by implementing the method of determination according to any of claim 1 to 14.

16. Enzymatic assay for *in vitro* testing compounds for their inhibitory effect on an enzymatic composition as disclosed to any one of claims 1 to 15, comprising :
(i) the substrate 7,8-dihydroneopterin, and
(ii) the Fol1 enzyme, and
(iii)the enzyme pyrophosphatase, and
(iv) pABA and ATP for simultaneous or sequential use.


SEQUENCE LISTING

<110> AVENTIS PHARMA S.A.

<120> METHOD FOR DETERMINING INHIBITION ACTIVITY OF A
      COMPOUND ON ONE OF THE ENZYMES OF THE FOLATE SYNTHESIS
      PATHWAY

<130> 18119RUU

<140>
<141>

<160> 2

<170> PatentIn Ver. 2.1

<210> 1
<211> 2364
<212> DNA
<213> Candida albicans

<220>
<221> CDS
<222> (1)..(2364)

<220>
<221> gene
<222> (1)..(2364)
<223> gene CaNL256

<400> 1
atg ttg aaa aac gat acc gtt ttc act aaa gat att tct tgt acg gcg    48
Met Leu Lys Asn Asp Thr Val Phe Thr Lys Asp Ile Ser Cys Thr Ala
  1               5                  10                  15

ata act ggt aaa gat gcc tgg aat cgg cca aca cca caa cca atc act    96
Ile Thr Gly Lys Asp Ala Trp Asn Arg Pro Thr Pro Gln Pro Ile Thr
             20                  25                  30

ata tca tta tct ttc aat act gat ttc cat aag gca tcg gaa ttg gat    144
Ile Ser Leu Ser Phe Asn Thr Asp Phe His Lys Ala Ser Glu Leu Asp
         35                  40                  45

aat ttg aaa tac tca att aat tat gct gtt att acc aga aat gta act    192
Asn Leu Lys Tyr Ser Ile Asn Tyr Ala Val Ile Thr Arg Asn Val Thr
     50                  55                  60

gaa ttt atg aaa tca aat gag cat tta aat ttc aag tca tta gga aat    240
Glu Phe Met Lys Ser Asn Glu His Leu Asn Phe Lys Ser Leu Gly Asn
65                  70                  75                  80

att gct caa gca att agt gat att gga tta gat caa tct aga ggt ggt    288
Ile Ala Gln Ala Ile Ser Asp Ile Gly Leu Asp Gln Ser Arg Gly Gly
                85                  90                  95

gga tct att gtg gat gtg acg ata aaa agt ttg aaa tca gaa ata aga    336
Gly Ser Ile Val Asp Val Thr Ile Lys Ser Leu Lys Ser Glu Ile Arg
            100                 105                 110

gct gaa agt gtc gaa tat aaa att aat aga aac act ttg ggt caa ccc    384
Ala Glu Ser Val Glu Tyr Lys Ile Asn Arg Asn Thr Leu Gly Gln Pro
        115                 120                 125

gtt cca tta gat att ttc caa gtt aat aaa ttg aga tta ttg acg att    432
Val Pro Leu Asp Ile Phe Gln Val Asn Lys Leu Arg Leu Leu Thr Ile
    130                 135                 140

att gga gtt ttc aca ttt gaa aga tta caa aaa caa ata gtt gat gtt    480
Ile Gly Val Phe Thr Phe Glu Arg Leu Gln Lys Gln Ile Val Asp Val
145                 150                 155                 160

gat ttg caa ttt aaa att gaa cct aat tcc aat tta tat ttc cat caa    528
Asp Leu Gln Phe Lys Ile Glu Pro Asn Ser Asn Leu Tyr Phe His Gln
                165                 170                 175

ata att gct gat att gtt tca tac gtg gaa tca tct aat ttc aaa act    576
Ile Ile Ala Asp Ile Val Ser Tyr Val Glu Ser Ser Asn Phe Lys Thr
            180                 185                 190

gta gaa gca ttg gtg tct aag att ggt caa ttg aca ttt cag aaa tat    624
Val Glu Ala Leu Val Ser Lys Ile Gly Gln Leu Thr Phe Gln Lys Tyr
        195                 200                 205

gac gga gta gct gaa gtt gtt gct act gtc act aaa ccg aat gca ttt    672
Asp Gly Val Ala Glu Val Val Ala Thr Val Thr Lys Pro Asn Ala Phe
    210                 215                 220

agt cat gtt gaa ggt gtt gga gta tca tct acc atg gtc aaa gac aat    720
Ser His Val Glu Gly Val Gly Val Ser Ser Thr Met Val Lys Asp Asn
225                 230                 235                 240

ttc aaa gat atg gaa cca gtt aaa ttt gaa aac aca att gct caa act    768
Phe Lys Asp Met Glu Pro Val Lys Phe Glu Asn Thr Ile Ala Gln Thr
                245                 250                 255

aat aga gca ttc aat tta cct gtt gaa aat gag aaa act gag gat tat    816
Asn Arg Ala Phe Asn Leu Pro Val Glu Asn Glu Lys Thr Glu Asp Tyr
            260                 265                 270

acc ggg tac cac act gca ttt att gcc ttt gga tcc aat act gga aat    864
Thr Gly Tyr His Thr Ala Phe Ile Ala Phe Gly Ser Asn Thr Gly Asn
        275                 280                 285

caa gta gaa aat att acc aat tca ttc gaa ttg ttg caa aaa tat gga    912
Gln Val Glu Asn Ile Thr Asn Ser Phe Glu Leu Leu Gln Lys Tyr Gly
    290                 295                 300

atc acc ata gaa gca act tca tca ttg tac att tct aaa cca atg tat    960
Ile Thr Ile Glu Ala Thr Ser Ser Leu Tyr Ile Ser Lys Pro Met Tyr
305                 310                 315                 320

tac ttg gat caa cca gat ttt ttc aat gga gta att aaa gtg aat ttc    1008
Tyr Leu Asp Gln Pro Asp Phe Phe Asn Gly Val Ile Lys Val Asn Phe
                325                 330                 335

caa aac att tca cct ttc cag ttg ttg aaa att cta aaa gat att gaa    1056
Gln Asn Ile Ser Pro Phe Gln Leu Leu Lys Ile Leu Lys Asp Ile Glu
            340                 345                 350

tat aaa cat tta gaa agg aaa aaa gac ttt gat aat ggg ccc aga tca    1104
Tyr Lys His Leu Glu Arg Lys Lys Asp Phe Asp Asn Gly Pro Arg Ser
        355                 360                 365

ata gat ttg gat att ata cta tat gac gat tta caa tta aat acc gag    1152
Ile Asp Leu Asp Ile Ile Leu Tyr Asp Asp Leu Gln Leu Asn Thr Glu
    370                 375                 380

aat cta att att cca cat aaa tca atg tta gaa aga aca ttt gta tta    1200
Asn Leu Ile Ile Pro His Lys Ser Met Leu Glu Arg Thr Phe Val Leu
385                 390                 395                 400

caa cca tta tgt gaa gta ttg ccc cct gat tat att cat ccc atc agt    1248
Gln Pro Leu Cys Glu Val Leu Pro Pro Asp Tyr Ile His Pro Ile Ser
                405                 410                 415

gca gaa agt ttg cat agc cat tta caa caa tta ata aat gat aaa cct    1296
Ala Glu Ser Leu His Ser His Leu Gln Gln Leu Ile Asn Asp Lys Pro
            420                 425                 430

caa gag aca gta caa gaa tcg tct gat tta tta caa ttt atc cca gtc    1344
Gln Glu Thr Val Gln Glu Ser Ser Asp Leu Leu Gln Phe Ile Pro Val
        435                 440                 445

tct aga ttg cct gtc aaa gat aat att ttg aaa ttt gat caa att aat    1392
Ser Arg Leu Pro Val Lys Asp Asn Ile Leu Lys Phe Asp Gln Ile Asn
    450                 455                 460

cat aaa tct cct act ttg att atg ggt ata ttg aat atg act cct gat    1440
His Lys Ser Pro Thr Leu Ile Met Gly Ile Leu Asn Met Thr Pro Asp
465                 470                 475                 480

tca ttt agt gat ggt ggg aaa cat ttt gga aaa gaa cta gat aat act    1488
Ser Phe Ser Asp Gly Gly Lys His Phe Gly Lys Glu Leu Asp Asn Thr
                485                 490                 495

gtg aag cag gca gag aaa tta gtc agt gag ggt gct acg att att gac    1536
Val Lys Gln Ala Glu Lys Leu Val Ser Glu Gly Ala Thr Ile Ile Asp
            500                 505                 510

att gga gga gtt tcc aca cgc cca gga agt gtt gaa ccc act gag gaa    1584
Ile Gly Gly Val Ser Thr Arg Pro Gly Ser Val Glu Pro Thr Glu Glu
        515                 520                 525

gaa gaa ttg gaa cgt gtg att cca tta att aaa gct att cgt caa tca    1632
Glu Glu Leu Glu Arg Val Ile Pro Leu Ile Lys Ala Ile Arg Gln Ser
    530                 535                 540

ctg aac cct gat tta ctg aag gtg ttg att tcg gtt gat act tat cgt    1680
Leu Asn Pro Asp Leu Leu Lys Val Leu Ile Ser Val Asp Thr Tyr Arg
545                 550                 555                 560

agg aac gtt gct gaa caa agt tta ctt gtg ggt gct gac ata atc aac    1728
Arg Asn Val Ala Glu Gln Ser Leu Leu Val Gly Ala Asp Ile Ile Asn
                565                 570                 575

gat atc tca atg ggc aaa tat gat gaa aaa ata ttt gat gtg gtt gct    1776
Asp Ile Ser Met Gly Lys Tyr Asp Glu Lys Ile Phe Asp Val Val Ala
            580                 585                 590

aaa tac gga tgt cct tat atc atg aat cat act cga gga tca cct aaa    1824
Lys Tyr Gly Cys Pro Tyr Ile Met Asn His Thr Arg Gly Ser Pro Lys
        595                 600                 605

acc atg tct aaa ttg acc aat tat gaa tca aat aca aat gat gat att    1872
Thr Met Ser Lys Leu Thr Asn Tyr Glu Ser Asn Thr Asn Asp Asp Ile
    610                 615                 620

atc gaa tat ata att gat cct aaa tta gga cat caa gaa ttg gat ttg    1920
Ile Glu Tyr Ile Ile Asp Pro Lys Leu Gly His Gln Glu Leu Asp Leu
625                 630                 635                 640

tca cct gaa atc aag aat tta ctc aat gga atc agt cgt gaa ttg agt 1968
Ser Pro Glu Ile Lys Asn Leu Leu Asn Gly Ile Ser Arg Glu Leu Ser
                645                 650                 655

tta caa atg ttt aaa gcc atg gct aaa gga gtg aaa aaa tgg caa att 2016
Leu Gln Met Phe Lys Ala Met Ala Lys Gly Val Lys Lys Trp Gln Ile
            660                 665                 670

att ttg gat cct ggt att gga ttt gct aaa aat ttg aat caa aat tta 2064
Ile Leu Asp Pro Gly Ile Gly Phe Ala Lys Asn Leu Asn Gln Asn Leu
        675                 680                 685

gca gtt att cgt aat gcc tcg ttt ttt aaa aaa tat tct att caa att 2112
Ala Val Ile Arg Asn Ala Ser Phe Phe Lys Lys Tyr Ser Ile Gln Ile
    690                 695                 700

aat gaa cgt gtt gat gat gtg aca atc aaa cat aaa tat tta agt ttt 2160
Asn Glu Arg Val Asp Asp Val Thr Ile Lys His Lys Tyr Leu Ser Phe
705                 710                 715                 720

aat ggt gct tgt gtt ttg gtg ggg aca tca aga aag aag ttt ttg ggg 2208
Asn Gly Ala Cys Val Leu Val Gly Thr Ser Arg Lys Lys Phe Leu Gly
                725                 730                 735

aca tta act ggt aat gaa gtg cct ctg gat cga gta ttt ggc act ggt 2256
Thr Leu Thr Gly Asn Glu Val Pro Leu Asp Arg Val Phe Gly Thr Gly
            740                 745                 750

gca aca gtg tct gcg tgt att gaa caa aac act gat att gta aga gtt 2304
Ala Thr Val Ser Ala Cys Ile Glu Gln Asn Thr Asp Ile Val Arg Val
        755                 760                 765

cat gat gtt aaa gaa atg aaa gat gta gta tgt ata agt gat gca att 2352
His Asp Val Lys Glu Met Lys Asp Val Val Cys Ile Ser Asp Ala Ile
    770                 775                 780

tat aaa aat gta 2364
Tyr Lys Asn Val
785

<210> 2
<211> 788
<212> PRT
<213> Candida albicans

<400> 2
Met Leu Lys Asn Asp Thr Val Phe Thr Lys Asp Ile Ser Cys Thr Ala

1               5                   10                  15

Ile Thr Gly Lys Asp Ala Trp Asn Arg Pro Thr Pro Gln Pro Ile Thr
            20                  25                  30

Ile Ser Leu Ser Phe Asn Thr Asp Phe His Lys Ala Ser Glu Leu Asp
        35                  40                  45

Asn Leu Lys Tyr Ser Ile Asn Tyr Ala Val Ile Thr Arg Asn Val Thr
    50                  55                  60

Glu Phe Met Lys Ser Asn Glu His Leu Asn Phe Lys Ser Leu Gly Asn
65                  70                  75                  80

Ile Ala Gln Ala Ile Ser Asp Ile Gly Leu Asp Gln Ser Arg Gly Gly
                85                  90                  95

Gly Ser Ile Val Asp Val Thr Ile Lys Ser Leu Lys Ser Glu Ile Arg
            100                 105                 110

Ala Glu Ser Val Glu Tyr Lys Ile Asn Arg Asn Thr Leu Gly Gln Pro
        115                 120                 125

Val Pro Leu Asp Ile Phe Gln Val Asn Lys Leu Arg Leu Leu Thr Ile
    130                 135                 140

Ile Gly Val Phe Thr Phe Glu Arg Leu Gln Lys Gln Ile Val Asp Val
145                 150                 155                 160

Asp Leu Gln Phe Lys Ile Glu Pro Asn Ser Asn Leu Tyr Phe His Gln
                165                 170                 175

Ile Ile Ala Asp Ile Val Ser Tyr Val Glu Ser Ser Asn Phe Lys Thr
            180                 185                 190

Val Glu Ala Leu Val Ser Lys Ile Gly Gln Leu Thr Phe Gln Lys Tyr
        195                 200                 205

Asp Gly Val Ala Glu Val Val Ala Thr Val Thr Lys Pro Asn Ala Phe
    210                 215                 220

Ser His Val Glu Gly Val Gly Val Ser Ser Thr Met Val Lys Asp Asn
225                 230                 235                 240

Phe Lys Asp Met Glu Pro Val Lys Phe Glu Asn Thr Ile Ala Gln Thr
                245                 250                 255

Asn Arg Ala Phe Asn Leu Pro Val Glu Asn Glu Lys Thr Glu Asp Tyr

            260                 265                 270

Thr Gly Tyr His Thr Ala Phe Ile Ala Phe Gly Ser Asn Thr Gly Asn
        275                 280                 285

Gln Val Glu Asn Ile Thr Asn Ser Phe Glu Leu Leu Gln Lys Tyr Gly
    290                 295                 300

Ile Thr Ile Glu Ala Thr Ser Ser Leu Tyr Ile Ser Lys Pro Met Tyr
305                 310                 315                 320

Tyr Leu Asp Gln Pro Asp Phe Phe Asn Gly Val Ile Lys Val Asn Phe
                325                 330                 335

Gln Asn Ile Ser Pro Phe Gln Leu Leu Lys Ile Leu Lys Asp Ile Glu
            340                 345                 350

Tyr Lys His Leu Glu Arg Lys Lys Asp Phe Asp Asn Gly Pro Arg Ser
        355                 360                 365

Ile Asp Leu Asp Ile Ile Leu Tyr Asp Asp Leu Gln Leu Asn Thr Glu
    370                 375                 380

Asn Leu Ile Ile Pro His Lys Ser Met Leu Glu Arg Thr Phe Val Leu
385                 390                 395                 400

Gln Pro Leu Cys Glu Val Leu Pro Pro Asp Tyr Ile His Pro Ile Ser
                405                 410                 415

Ala Glu Ser Leu His Ser His Leu Gln Gln Leu Ile Asn Asp Lys Pro
            420                 425                 430

Gln Glu Thr Val Gln Glu Ser Ser Asp Leu Leu Gln Phe Ile Pro Val
        435                 440                 445

Ser Arg Leu Pro Val Lys Asp Asn Ile Leu Lys Phe Asp Gln Ile Asn
    450                 455                 460

His Lys Ser Pro Thr Leu Ile Met Gly Ile Leu Asn Met Thr Pro Asp
465                 470                 475                 480

Ser Phe Ser Asp Gly Gly Lys His Phe Gly Lys Glu Leu Asp Asn Thr
                485                 490                 495

Val Lys Gln Ala Glu Lys Leu Val Ser Glu Gly Ala Thr Ile Ile Asp
            500                 505                 510

Ile Gly Gly Val Ser Thr Arg Pro Gly Ser Val Glu Pro Thr Glu Glu

        515                 520                 525

Glu Glu Leu Glu Arg Val Ile Pro Leu Ile Lys Ala Ile Arg Gln Ser
    530                 535                 540

Leu Asn Pro Asp Leu Leu Lys Val Leu Ile Ser Val Asp Thr Tyr Arg
545                 550                 555                 560

Arg Asn Val Ala Glu Gln Ser Leu Leu Val Gly Ala Asp Ile Ile Asn
                565                 570                 575

Asp Ile Ser Met Gly Lys Tyr Asp Glu Lys Ile Phe Asp Val Val Ala
            580                 585                 590

Lys Tyr Gly Cys Pro Tyr Ile Met Asn His Thr Arg Gly Ser Pro Lys
        595                 600                 605

Thr Met Ser Lys Leu Thr Asn Tyr Glu Ser Asn Thr Asn Asp Asp Ile
    610                 615                 620

Ile Glu Tyr Ile Ile Asp Pro Lys Leu Gly His Gln Glu Leu Asp Leu
625                 630                 635                 640

Ser Pro Glu Ile Lys Asn Leu Leu Asn Gly Ile Ser Arg Glu Leu Ser
                645                 650                 655

Leu Gln Met Phe Lys Ala Met Ala Lys Gly Val Lys Lys Trp Gln Ile
            660                 665                 670

Ile Leu Asp Pro Gly Ile Gly Phe Ala Lys Asn Leu Asn Gln Asn Leu
        675                 680                 685

Ala Val Ile Arg Asn Ala Ser Phe Phe Lys Lys Tyr Ser Ile Gln Ile
    690                 695                 700

Asn Glu Arg Val Asp Asp Val Thr Ile Lys His Lys Tyr Leu Ser Phe
705                 710                 715                 720

Asn Gly Ala Cys Val Leu Val Gly Thr Ser Arg Lys Lys Phe Leu Gly
                725                 730                 735

Thr Leu Thr Gly Asn Glu Val Pro Leu Asp Arg Val Phe Gly Thr Gly
            740                 745                 750

Ala Thr Val Ser Ala Cys Ile Glu Gln Asn Thr Asp Ile Val Arg Val
        755                 760                 765

His Asp Val Lys Glu Met Lys Asp Val Val Cys Ile Ser Asp Ala Ile

    770                 775                 780

Tyr Lys Asn Val
785